(19) Europäisches Patentamt
European Patent Office
Office européen des brevets

(11) Veröffentlichungsnummer: **0 545 861 B1**

# (12) EUROPÄISCHE PATENTSCHRIFT

(45) Veröffentlichungstag der Patentschrift: **27.12.95**

(21) Anmeldenummer: **92810904.0**

(22) Anmeldetag: **20.11.92**

(51) Int. Cl.$^6$: **C07C 235/34**, C07C 69/734, C07C 59/64, C07C 59/66, C09K 15/22, C09K 15/08, C08K 5/10, C08K 5/20

## (54) Diphenylessigsäurederivate als Stabilisatoren für organisches Material

(30) Priorität: **29.11.91 CH 3501/91**

(43) Veröffentlichungstag der Anmeldung:
**09.06.93 Patentblatt 93/23**

(45) Bekanntmachung des Hinweises auf die Patenterteilung:
**27.12.95 Patentblatt 95/52**

(84) Benannte Vertragsstaaten:
**DE FR GB IT**

(56) Entgegenhaltungen:
**EP-A- 0 415 887**
**WO-A-80/01566**

(73) Patentinhaber: **CIBA-GEIGY AG**
**Klybeckstrasse 141**
**CH-4002 Basel (CH)**

(72) Erfinder: **Nesvadba, Peter, Dr.**
**Route du Nord 5**
**Ch-1723 Marly (CH)**

EP 0 545 861 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Diphenylessigsäurederivate, Zusammensetzungen, welche diese enthalten, sowie die Verwendung dieser Derivate als Stabilisatoren für organisches Material.

Es ist bekannt, organischen Materialien bestimmte Chemikalien als Stabilisatoren zuzusetzen. Dazu gehören die dem Fachmann wohlbekannten Antioxidantien und Verarbeitungsstabilisatoren.

Es besteht weiterhin ein Bedarf an solchen Zusatzstoffen.

Es wurde nun überraschend gefunden, daß die unten beschriebenen Verbindungen der Formel I sich ausgezeichnet als Additive zur Verhinderung des lichtinduzierten, thermischen oder/und oxidativen Abbaus organischer Materialien eignen.

Die Erfindung betrifft daher Verbindungen der Formel (I)

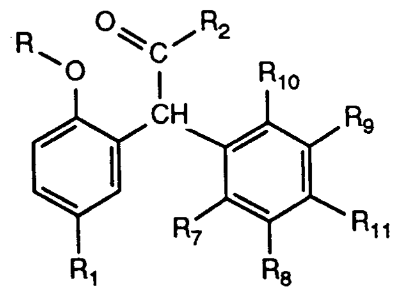

(I)

worin R unsubstituiertes oder mit Phenyl substituiertes $C_3$-$C_{18}$-2-Alkenyl,
$C_3$-$C_8$-2-Alkinyl oder Phenyl-$C_1$-$C_4$-alkyl ist,
$R_1$ Wasserstoff, $C_1$-$C_{18}$-Alkyl, $C_5$-$C_{12}$-Cycloalkyl oder Phenyl-$C_1$-$C_4$-alkyl darstellt,
$R_2$ -$OR_4$ oder -$NR_5R_6$ ist,
$R_4$ Wasserstoff, $C_1$-$C_{18}$-Alkyl oder $(-CH_2-)_nG$ darstellt,
$R_5$ und $R_6$ unabhängig voneinander Wasserstoff, $C_1$-$C_{12}$-Alkyl oder Phenyl-$C_1$-$C_4$-alkyl bedeuten,
$R_7$, $R_8$, $R_9$ und $R_{10}$ unabhängig voneinander Wasserstoff, $C_1$-$C_4$-Alkyl oder $C_1$-$C_4$-Alkoxy darstellen, mit der Bedingung, dass mindestens einer der Reste $R_7$, $R_8$, $R_9$ oder $R_{10}$ Wasserstoff ist,
$R_{11}$ Wasserstoff, $C_1$-$C_{18}$-Alkoxy, $C_5$-$C_8$-Cycloalkoxy, Phenyl-$C_1$-$C_4$-alkoxy oder
$C_3$-$C_{18}$-2-Alkenyloxy bedeutet,
n eine Zahl von 2 bis 12 ist, und
G eine Gruppe der Formel

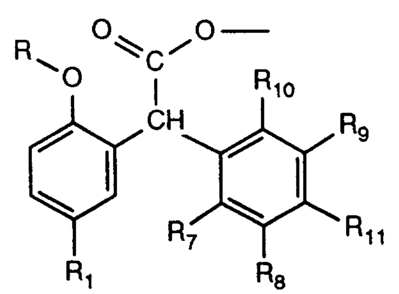

darstellt.

R als unsubstituiertes oder mit Phenyl substituiertes $C_3$-$C_{18}$-2-Alkenyl kann verzweigt oder unverzweigt sein und beispielsweise Allyl, 2-Methallyl, 2-Hexenyl, 2-Dodecenyl, 2-Tetradecenyl oder 3-Phenyl-2-propenyl bedeuten.

R leitet sich als $C_3$-$C_8$-2-Alkinyl von den Alkylresten mit 3 bis 8 C-Atomen ab, in denen 2 C-Atome durch eine Dreifachbindung verbunden sind, und ist bevorzugt Propargyl.

R, $R_1$, $R_5$ und $R_6$ als Phenyl-$C_1$-$C_4$-alkyl bedeutet z.B. Benzyl, Phenethyl, 3-Phenylpropyl, α-Methylbenzyl oder α,α-Dimethylbenzyl. Bevorzugt ist Benzyl.

Stellen in obiger Formel $R_1$, $R_4$, $R_3$, $R_5$ und $R_6$ $C_1$-$C_{18}$-Alkyl oder $C_1$-$C_{12}$-Alkyl dar, so handelt es sich dabei um verzweigte oder unverzweigte Reste. Beispiele hierfür sind Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl.

$R_1$ als $C_5$-$C_{12}$-Cycloalkyl kann z.B. Cyclopentyl, Cyclohexyl, Cycloheptyl, Cyclooctyl oder Cyclododecyl sein. Cyclopentyl und Cyclohexyl ist bevorzugt, insbesondere Cyclohexyl.

Stellen in obiger Formel $R_7$, $R_8$, $R_9$, $R_{10}$ und $R_{11}$ $C_1$-$C_4$-Alkoxy oder $C_1$-$C_{18}$-Alkoxy dar, so handelt es sich dabei um verzweigte oder unverzweigte Reste. Beispiele dafür sind Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy.

$R_{11}$ als $C_5$-$C_8$-Cycloalkoxy bedeutet beispielsweise Cyclopentoxy, Cyclohexoxy, Cycloheptoxy oder Cyclooctoxy.

$R_{11}$ als Phenyl-$C_1$-$C_4$-alkoxy bedeutet beispielsweise Benzyloxy, Phenethoxy, 3-Phenylpropoxy, $\alpha$-Methylbenzyloxy oder $\alpha,\alpha$-Dimethylbenzyloxy. Bevorzugt ist Benzyloxy.

$R_{11}$ als $C_3$-$C_{18}$-2-Alkenyloxy kann verzweigt oder unverzweigt sein und beispielsweise Allyloxy, 2-Methallyloxy, 2-Hexenyloxy, 2-Dodecenyloxy, 2-Tetradecenyloxy oder 3-Phenyl-2-propenyloxy bedeuten.

Zweckmäßig sind Verbindungen der Formel I, worin $R_7$, $R_8$, $R_9$, $R_{10}$ und $R_{11}$ Wasserstoffsind.

Bevorzugt sind Verbindungen der Formel I, worin

R $-CH_2-CH=CH_2$, $-CH_2-C(CH_3)=CH_2$, $-CH_2-CH=CHR_3$, $-CH_2-C\equiv CH$ oder Phenyl-$C_1$-$C_4$-alkyl ist,

$R_1$ Wasserstoff oder $C_1$-$C_8$-Alkyl bedeutet,

$R_2$ $-OR_4$ oder $-NHR_5$ ist,

$R_3$ Phenyl, $C_1$-$C_{12}$-Alkyl, und

$R_5$ Wasserstoff, $C_1$-$C_4$-Alkyl oder Benzyl bedeutet, und

$R_{11}$ Wasserstoff, $C_1$-$C_{18}$-Alkoxy, $C_3$-$C_4$-2-Alkenyloxy oder Benzyloxy bedeutet.

Besonders bevorzugt sind Verbindungen der Formel I, worin $R_1$ Methyl, t-Butyl oder $-C(CH_3)_2-CH_2-C-(CH_3)_3$ bedeutet.

Von besonderem Interesse sind Verbindungen der Formel I, worin $R_7$ und $R_{10}$ Wasserstoff sind.

Speziell bevorzugt sind Verbindungen der Formel I, worin

R unsubstituiertes oder mit Phenyl substituiertes $C_3$-$C_{14}$-2-Alkenyl, $-CH_2-C\equiv CH$ oder Benzyl bedeutet,

$R_1$ Wasserstoff oder $C_1$-$C_8$-Alkyl darstellt,

$R_2 OR_4$ oder $NH_2$ ist,

$R_7$ und $R_{10}$ Wasserstoff bedeuten,

$R_8$ und $R_9$ unabhängig voneinander Wasserstoff oder $C_1$-$C_4$-Alkyl darstellen, und

$R_{11}$ Wasserstoff oder $C_1$-$C_4$-Alkoxy bedeutet.

Die Verbindungen der Formel I eignen sich hervortagend zum Stabilisieren von organischen Materialien gegen lichtinduzierten, thermischen oder/und oxidativen Abbau. Gegenstand der Erfindung sind daher auch Zusammensetzungen enthaltend ein gegen solche Abbaureaktionen empfindliches organisches Material und mindestens eine Verbindung der Formel I bzw. die Verwendung von Verbindungen der Formel I als Stabilisatoren für organische Materialien gegen die genannten Abbauaarten.

Die Verbindungen der Formel I können insbesondere als Stabilisatoren für natürliche, halbsynthetisehe oder synthetische Polymere, besonders thermoplastische Kunststoffe (vor allem Polyolefine) und Elastomere, sowie für funktionelle Flüssigkeiten, insbesondere Schmierstoffe und Hydraulikflüssigkeiten, eingesetzt werden. Beispiele für solche Substrate sind der folgenden Aufzählung von geeigneten Materialien zu entnehmen.

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), PropylenButen-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

3a. Kohlenwasserstoffharze (z.B. $C_5$-$C_9$) inklusive hydrierte Modifikationen davon (z.B. Klebrigmacherharze) und Mischungen von Polyalkylenen und Stärke.

4. Polystyrol, Poly-(p-methylstyrol), Poly-(α-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner

mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

In den erfindungsgemäßen Zusammensetzungen sind die Verbindungen der Formel I zweckmäßig zu 0,0005 bis 5, beispielsweise zu 0,05 bis 5, vorzugsweise zu 0,05 bis 3, insbesondere jedoch zu 0,1 bis 2 Gew.-% enthalten. Es kann sich dabei um eine oder mehrere dieser Verbindungen der Formel I handeln, und die Gewichtsprozentangaben beziehen sich auf die gesamte Menge dieser Verbindungen. Berechnungsgrundlage ist dabei das Gesamtgewicht des organischen Materials ohne die Verbindungen der Formel I.

Die Einarbeitung in die Materialien kann beispielsweise durch Einmischen oder Aufbringen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach der Polymerisation der entsprechenden Monomeren bzw. vor der Vernetzung. Die Verbindungen der Formel I können dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulatoren für die Kettenlänge der Polymeren (Kettenabbrecher) wirken.

Die Verbindungen der Formel I oder Mischungen davon können auch in Form eines Masterbatches, der diese Verbindungen beispielsweise in einer Konzentration von 2.5 bis 25 Gew.-% enthält, den zu stabilisierenden Kunststoffen zugesetzt werden.

Zweckmäßig kann die Einarbeitung der Verbindungen der Formel I nach folgenden Methoden erfolgen:

- als Emulsion oder Dispersion (z.B. zu Latices oder Emulsionspolymeren)

- Als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Die Erfindung betrifft auch ein Verfahren zum Stabilisieren von organischem Material, insbesondere von thermoplastischen Polymeren (vor allem von Polyolefinen), Elastomeren oder funktionellen Flüssigkeiten gegen oxidativen, thermischen und/oder lichtinduzierten Abbau, dadurch gekennzeichnet, daß man diesem Material als Stabilisatoren Verbindungen der Formel I zusetzt bzw. auf dieses aufbringt.

Zusätzlich zu den erfindungsgemäßen Verbindungen bzw. Mischungen können die erfindungsgemäßen Zusammensetzungen, insbesondere wenn sie organische, vorzugsweise synthetische, Polymere enthalten, noch weitere übliche Additive enthalten. Beispiele für solche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Di-methyl-6-(1'-methyl-tridec-1'-yl)-phenol und Mischungen davon.

1.2. Alkylthiomethylphenole, z.B. 2,4-Di-octylthiomethyl-6-tert.-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol, 2,6-Di-tert.-butyl-hydrochinon, 2,5-Di-tert.-butyl-4-hydroxyanisol,3,5-Di-tert.-butyl-4-hydroxyanisol, 3,5-Di-tert.-butyl-4-hydroxyphenyl-stearat, Bis-(3,5-di-tert.-butyl-4-hydroxyphenyl)adipat.

1.4. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol), 4,4'-Thio-bis-(3,6-di-sec.-amylphenol), 4,4'-Bis-(2,6-dimethyl-4-hydroxyphenyl)-disulfid.

1.5. Alkyliden-Bisphenole, z.B. 2,2'-Methylen-bis-(6-tert.butyl-4-methylphenol), 2,2'-Methylen-bis-(6-tert.butyl-4-ethylphenol),2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.buty-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat, 1,1-Bis-(3,5-dimethy-2-hydroxyphenyl)-butan, 2,2-Bis-(3,5-di-tert.-butyl-4-hydroxyphenyl)-propan, 2,2-Bis-(5-tert.-butyl-4-hydroxy-2-methylphenyl)-4-n-dodecylmercapto-butan, 1,1,5,5-Tetra-(5-tert.-butyl-4-hydroxy-2-methylphenyl)-pentan.

1.6. O-, N- und S-Benzylverbindungen, z.B. 3,5,3',5'-Tetra-tert.-butyl-4,4'-dihydroxydibenzylether, Octadecyl-4-hydroxy-3,5-dimethylbenzyl-mercaptoacetat, Tris-(3,5-di-tert.-butyl-4-hydroxybenzyl)-amin, Bis-(4-tert.-butyl-3-hydroxy-2,6-dimethylbenzyl)-dithioterephthalat, Bis-(3,5-di-tert.-butyl-4-hydroxybenzyl)-sulfid, Isooctyl-3,5-di-tert.-butyl-4-hydroxybenzyl-mercaptoacetat.

1.7. Hydroxybenzylierte Malonate, z.B. Dioctadecyl-2,2-bis-(3,5-di-tert.-butyl-2-hydroxybenzyl)-malonat, Di-octadecyl-2-(3-tert.-butyl-4-hydroxy-5-methylbenzyl)-malonat, Di-dodecylmercaptoethyl-2,2-bis-(3,5-di-tert.-butyl-4-hydroxybenzyl)-malonat, Di-[4-(1,1,3,3-tetramethylbutyl)-phenyl]-2,2-bis-(3,5-di-tert.-butyl-4-hydroxybenzyl)-malonat.

1.8. Hydroxybenzyl-Aromaten, z.B. 1,3,5-Tris-(3,5-di-tert.-butyl-4-hydroxybenzyl)-2,4,6-trimethylbenzol, 1,4-Bis-(3,5-di-tert.-butyl-4-hydroxybenzyl)-2,3,5,6-tetramethylbenzol, 2,4,6-Tris-(3,5-di-tert.-butyl-4-hydroxybenzyl)-phenol.

1.9. Triazinverbindungen, z.B. 2,4-Bis-octylmercapto-6-(3,5-di-tert.-butyl-4-hydroxyanilino)-1,3,5-triazin, 2-Octylmercapto-4,6-bis-(3,5-di-tert.-butyl-4-hydroxyanilino)-1,3,5-triazin, 2-Octylmercapto-4,6-bis-(3,5-di-tert.-butyl-4-hydroxyphenoxy)-1,3,5-triazin, 2,4,6-Tris-(3,5-di-tert.-butyl-4-hydroxyphenoxy)-1,2,3-triazin, 1,3,5-Tris-(3,5-di-tert.-butyl-4-hydroxybenzyl)-isocyanurat, 1,3,5-Tris-(4-tert.-butyl-3-hydroxy-2,6-dimethylbenzyl)-isocyanurat, 2,4,6-Tris-(3,5-di-tert.-butyl-4-hydroxyphenylethyl)-1,3,5-triazin,1,3,5-Tris-(3,5-di-tert.butyl-4-hydroxyphenylpropionyl)-hexahydro-1,3,5-triazin, 1,3,5-Tris-(3,5-dicyclohexyl-4-hydroxybenzyl)-isocyanurat.

1.10. Benzylphosphonate, z.B. Dimethyl-2,5-di-tert.-butyl-4-hydroxybenzylphosphonat, Diethyl-3,5-di-tert.-butyl-4-hydroxybenzylphosphonat, Dioctadecyl-3,5-di-tert.-butyl-4-hydroxybenzylphosphonat, Dioctadecyl-5-tert.-butyl-4-hydroxy-3-methylbenzylphosphonat, Ca-Salz des 3,5-Di-tert.-butyl-4-hydroxybenzyl-phosphonsäure-monoethylesters.

1.11. Acylaminophenole, z.B. 4-Hydroxy-laurinsäureanilid, 4-Hydroxystearinsäureanilid, N-(3,5-di-tert.butyl-4-hydroxyphenyl)-carbaminsäureoctylester.

1.12. Ester der β-(3,5-Di-tert.butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.13. Ester der β-(5-tert.Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert.-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.-butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. 5'-Methyl-, 3',5',-Di-tert.butyl-, 5'-tert.-Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-, Mischung aus 5-Chlor-3'-tert.-butyl-5'-(2-octyloxycarbonylethyl)- und 5-Chlor-3'-tert.-butyl-5'-[2-(2-ethylhexyloxy)-carbonylethyl]-,5-Chlor-3'-tert.-butyl-5'-(2-methoxycarbonylethyl)-, 3'-tert.-Butyl-5'-(2-methoxycarbonylethyl)-, 3'-tert.-Butyl-5'-(2-octyloxycarbonylethyl)-, 3'-tert.-Butyl-5'-[2-(2-ethylhexyloxy)-carbonylethyl]-, 3'-Dodecyl-5'-methyl- und 3'-tert.-Butyl-5'-(2-isooctyloxycarbonylethyl)-2'-hydroxyphenyl-2H-benztriazol(2), 2,2'-Methylen-bis[4-(1,1,3,3-tetramethylbutyl)-6-benztriazol-2-yl-phenol]; Umesterungsprodukt von 2-[3'-tert.-Butyl-5'-(2-methoxycarbonylethyl)-2'-hydroxy-phenyl]-2H-benztriazol mit Polyethylenglycol 300; $[R-CH_2CH_2-COO(CH_2)_3]_2$ mit R = 3'-tert.-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butyl-benzoyl)-resorcin, Benzoylresorcin, 3,5-Di-tert.butyl-4-hydroxybenzoesäure-2,4-di-tert.-butylphenylester, 3,5-Di-tert.butyl-4-hydroxybenzoesäurehexadecylester, 3,5-Di-tert.-butyl-4-hydroxybenzoesäure-octadecylester, 3,5-Di-tert.-butyl-4-hydroxybenzoesäure-2-methyl-4,6-di-tert.-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch behinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert.butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethylpiperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert.-butyl-benzyl)-malonat, 3-n-Octyl-7,7,9,9-tetramethyl-1,3,8-triazaspiro[4.5]-decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetal-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Bis-(2,6-di-tert.-buty-4-methylphenyl)-pentaerythritdiphosphit, Bis-isodecyloxy-pentaerythritdiphosphit, Bis-(2,4-di-tert.-butyl-6-methylphenyl)-pentaerythritdiphosphit, Bis-(2,4,6-tri-tert.-butylphenyl)-pentaerythritdiphosphit, Tristearyl-sorbit-triphosphit, Tetrakis-(2,4-di-tert.butylphenyl)-4,4'-biphenylen-diphosphonit, 6-Isooctyloxy-2,4,8,10-tetra-tert.-butyl-12H-dibenz[d,g]-1,3,2-dioxaphosphocin, 6-Fluor-2,4,8,10-tetra-tert.-butyl-12-methyl-dibenz[d,g]-1,3,2-dioxaphosphocin.

5. Peroxidzerstörende Verbindungen, wie z.B. Ester der $\beta$-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-($\beta$-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Costabilisatoren werden beispielsweise in Konzentrationen von 0,01 bis 10 %, bezogen auf das Gesamtgewicht des zu stabilisierenden Materials, zugesetzt.

Die erfindungsgemässen Verbindungen der Formel I können insbesondere zusammen mit phenolischen Antioxidantien eingesetzt werden. Die erfindungsgemässen Zusammensetzungen enthalten daher vorzugsweise neben Verbindungen der Formel I phenolische Antioxidantien, insbesondere solche, wie sie in den obigen Punkten 1.1 bis 1.16 aufgelistet sind.

Andere bevorzugte Zusammensetzungen enthalten neben den Verbindungen der Formel I mindestens ein organisches Phosphit oder Phosphonit.

Wie bereits hervorgehoben, werden die erfindungsgemässen Verbindungen besonders vorteilhaft als Stabilisatoren in Polyolefinen eingesetzt, vor allem als Thermostabilisatoren. Ausgezeichnete Stabilisierung wird z.B. dann erhalten, wenn man sie in Kombination mit organischen Phosphiten oder Phosphoniten einsetzt. Dabei weisen die erfindungsgemässen Verbindungen den Vorteil auf, dass sie bereits in ausserordentlich geringen Mengen wirksam sind. Sie werden z.B. in Mengen von 0,0001 bis 0,015, insbesondere 0,0001 bis 0,008 Gew. % bezogen auf das Polyolefin, eingesetzt. Das organische Phosphit oder Phosphonit wird zweckmässig in einer Menge von 0,01 bis 2, insbesondere 0,01 bis 1 Gew. %, ebenfalls bezogen auf das Polyolefin, eingesetzt. Als organische Phosphite bzw. Phosphonite werden vorzugsweise solche eingesetzt, wie sie in der deutschen Patentanmeldung P 4202276.2 beschrieben sind. Siehe dort insbesondere die Patentansprüche, die Beispiele sowie die Seiten 5, letzter Absatz bis Seite 11. Besonders zweckmässige Phosphite und Phosphonite sind auch Punkt 4 der obigen Auflistung von Costabilisatoren zu entnehmen.

Die vorliegende Erfindung betrifft auch die Verwendung von Verbindungen der Formel I zum Stabilisieren von gegen oxidativen, thermischen und/oder lichtinduzierten Abbau empfindlichem organischem Material, insbesondere von natürlichen oder (halb)synthetischen Polymeren, vor allem von thermoplastischen Polymeren oder Elastomeren und ganz besonders als Verarbeitungsstabilisatoren für thermoplastische Polymere.

Zweckmäßige und bevorzugte Verbindungen der Formel I, wie vorstehend beschrieben führen zu zweckmäßigen und bevorzugten Zusammensetzungen.

Die Herstellung der erfindungsgemäßen Verbindungen geht z.B. von Benzofuranonen der Formel II aus, deren Herstellung in US-A-4,325,863 und US-A-4,338,244 beschrieben ist. Umsetzung mit Ammoniak oder primären oder sekundären Aminen $HNR_5R_6$ führt zu Amiden der Formel III, die sich mit organischen Halogeniden R-Hal (Hal = Halogen, bevorzugt Chlor) zu Amiden der Formel IV umsetzen lassen. Diese können z.B. mit Basen oder Mineralsäuren in Säuren der Formel V umgewandelt werden, welche sich nach bekannten Methoden (z.B. durch Umsetzung mit Alkoholen der Formel $R_4OH$ mit $R_4 \neq H$) zu Verbindungen der Formel I mit $R_2 = OR_4$ ($R_4 \neq H$) verestern lassen.

(II) 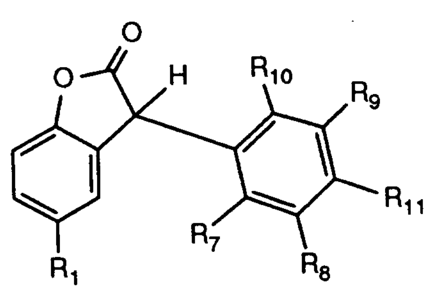 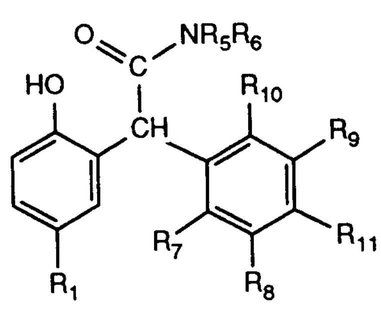 (III)

(IV) 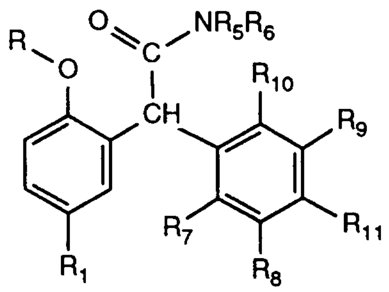 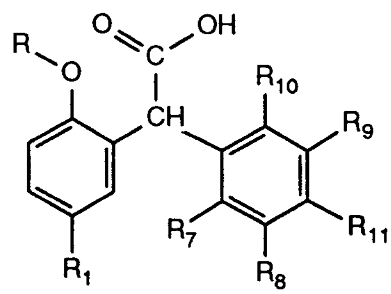 (V)

Die Verbindungen der Formel II können wie erwähnt auf an sich bekannte Weise hergestellt werden.

Beispielsweise, und dies ist bevorzugt, wird ein Phenol der Formel VI,

(VI) 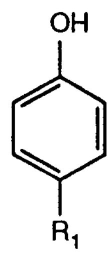 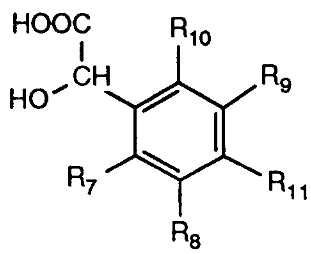 (VII)

worin $R_1$ die angegebene Bedeutung hat, mit einem am Phenylring substituierten Mandelsäure-Derivat der Formel VII, worin $R_7$, $R_8$, $R_9$, $R_{10}$ und $R_{11}$ die angegebenen Bedeutungen haben, bei erhöhter Temperatur, insbesondere Temperaturen von 130 bis 200 °C in der Schmelze oder in einem Lösungsmittel gegebenenfalls unter leichtem Vakuum, zu Verbindungen der Formel II umgesetzt. Bevorzugt wird die Reaktion in einem Lösungsmittel wie beispielsweise Essigsäure, Propionsäure oder Ameisensäure in einem Temperaturbereich von 50 bis 130 °C durchgeführt. Die Reaktion kann durch Zusatz einer Säure wie Salzsäure, Schwefelsäure oder Methansulfonsäure katalysiert werden.

Die am Phenylring substituierten Mandelsäuren der Formel VII sind in der Literatur bekannt oder können beispielsweise gemäss W. Bradley et al, J. Chem. Soc. 1956, 1622; EP-A-146269, EP-B-182507 (Beispiel 1, Seite 4) oder DE-A-2 944 295 in analoger Weise hergestellt werden.

Die folgenden Beispiele erläutern die Erfindung weiter. Angaben in Teilen oder Prozenten beziehen sich auf das Gewicht.

Beispiel 1: Herstellung von 2-(2-Allyloxy-5-tert.butyl-phenyl)-2-phenylessigsäureamid (Verbindung (101), Tabelle 1).

Eine Lösung von 53,26 g (0,20 Mol) 5-tert.-Butyl-3-phenyl-benzofuran-2-on in 100 ml Tetrahydrofuran wird mit 18 ml 25 % wässriger Ammoniak-Lösung versetzt und während ca. 2,5 Stunden bei Raumtemperatur gerührt. Anschliessend werden 600 ml Wasser zugegeben und der ausgefallene Niederschlag filtriert. Der Rückstand wird mit Wasser gewaschen und getrocknet. Es resultieren 56,7 g (20 %) 2-(2-Hydroxy-5-tert.-butyl-phenyl)-2-phenylessigsäureamid.

Zu einer Natriumethylat-Lösung, hergestellt durch Zugabe von 2,3 g (100 mMol) Natrium in 200 ml absolutem Ethanol, werden 23,6 g (83 mMol) 2-(2-Hydroxy-5-tert.-butyl-phenyl)-2-phenylessigsäureamid gegeben und nachdem alles gelöst ist mit 13,8 g (110 mMol) Allylbromid versetzt und anschliessend während ca. 4,5 Stunden bei 50 °C gehalten. Das Reaktionsgemisch wird am Vakuumrotationsverdampfer eingeengt, der Rückstand in Methylenchlorid aufgenommen, filtriert und erneut am Vakuumrotationsverdampfer eingeengt. Kristallisation des Rückstandes aus Ethylacetat/Ligroin (1:1) liefert 22,3 g (83 %) 2-(2-Allyloxy-5-tert.-butyl-phenyl)-2-phenylessigsäureamid, Smp. 172-174 °C (Verbindung (101), Tabelle 1).

In Analogie zu Beispiel 1 werden aus den entsprechenden Benzofuran-2-onen der Formel II, wie beispielsweise 5-(1,1,3,3-Tetramethylbutyl)-3-phenyl-benzofuran-2-on, 3-(4-Ethoxy-phenyl)-5-methyl-benzofuran-2-on oder 5-tert.-Butyl-3-(3,5-dimethyl-4-methoxy-phenyl)-benzofuran-2-on, und Alkylhalogeniden, wie beispielsweise 3-Brom-1-phenyl-1-propen, Benzylchlorid, $CH_3(CH_2)_{10}$-CH = CH-$CH_2$Br [hergestellt aus $CH_3$-

$(CH_2)_{10}$-CH = CH-$CH_3$ mit N-Bromsuccinimid in Tetrachlorkohlenstoff] oder Propargylchlorid, die Verbindungen (102), (103), (104), (108), (109), (111) und (112) hergestellt.

Herstellung von Benzofuran-2-onen der Formel II:

Ein Gemisch von 1,50 Mol eines Phenols, wie beispielsweise 4-tert.-Butyl-phenol, 4-(1,1,3,3-Tetramethylbutyl)-phenol oder 4-Methyl-phenol,und 1,0 Mol einer Mandelsäure wird unter Stickstoffatmosphäre während 2 Stunden bei 140-150 °C gerührt. Anschliessend wird unter leichtem Vakuum (50 mbar) noch während 1,5 Stunden bei 150 °C nachgerührt. Das überschüssige Phenol wird am Hochvakuum abdestilliert Kristallisation des Rückstandes aus Xylol/Ethanol liefert die Benzofuran-2-one der Formel II wie beispielsweise 5-tert.-Butyl-3-phenyl-benzofuran-2-on, Smp. 134-135 °C (84 %), 5-(1,1,3,3-Tetramethylbutyl)-3-phenyl-benzofuran-2-on, Harz (Chromatographie an Kieselgel mit dem Laufmittelsystem Dichlormethan/Hexan 1:1) (46 %), 3-(4-Ethoxy-phenyl)-5-methylbenzofuran-2-on, Smp. 80-83 °C (59 %) oder 5-tert.-Butyl-3-(3,5-dimethyl-4-methoxyphenyl)-benzofuran-2-on, Smp. 121-123 °C (44 %).

Herstellung von substituierten Mandelsäuren:

20,8 g (0,10 Mol) 4-Hydroxymandelsäure-Natriumsalz-Monohydrat und 6,6 g (0,10 Mol) Kaliumhydroxid werden mit 1,0 g (6,7 mMol) Natriumiodid in 75 ml Methanol gelöst. Danach werden 0,12 Mol Alkylbromid (im Falle des Methallyls wird Methallylchlorid verwendet) zugegeben und unter Stickstoffatmosphäre während 16 Stunden unter Rückfluss gekocht. Das Reaktionsgemisch wird am Vakuumrotationsverdampfer eingeengt und der Rückstand mit konzentrierter Salzsäure angesäuert. Das Produkt wird mit Butylacetat dreimal extrahiert. Die organischen Phasen werden mit Wasser gewaschen, vereinigt, über Natriumsulfat getrocknet und am Vakuumrotationsverdampfer eingeengt Kristallisation des Rückstandes aus Toluol/Benzin liefert die 4-Alkoxy-mandelsäuren, wie z.B. 4-Methallyloxy-mandelsäure, Smp. 121-126 ° (65 %); 4-n-Tetradecyloxy-mandelsäure, Smp. 104-107 °C (68 %); 4-n-Octoxy-mandelsäure, Smp. 96-99 °C (58 %); 4-n-Hexoxy-mandelsäure, Smp. 103-106 °C (69 %); 4-n-Octadecyloxy-mandelsäure, Smp. 103-109 °C (29 %), 4-n-Butoxy-mandelsäure, Smp. 132-134 °C (67 %) oder 4-Ethoxymandelsäure, Smp. 127-129 °C (89%).

Herstellung von 4-Benzyloxy-mandelsäure:

Eine Lösung von 41,6 g (0,20 Mol) 4-Hydroxymandelsäure-Natriumsalz-Monohydrat, 9,6 g (0,24 Mol) Natriumhydroxid und 27,9 g (0,22 Mol) Benzylchlorid in 50 ml Wasser wird während 17 Stunden bei 70 °C gerührt. Anschliessend wird mit 50 ml Wasser verdünnt und nochmals 4,0g (0,10 Mol) Natriumhydroxid zugegeben. Das Reaktionsgemisch wird während einer Stunde am Rückfluss gekocht, dann abgekühlt, mit konzentrierter Salzsäure angesäuert und das ausgefallene Produkt filtriert. Der Rückstand wird mit kaltem Wasser gewaschen und anschliessend am Hochvakuum getrocknet. Es resultieren 35,6 g (69 %) 4-Benzyloxy-mandelsäure, Smp. 148-155 °C.

Herstellung von 4-Alkoxy-3,5-dimethyl-mandelsäuren:

Zu einer bei 100 °C rührenden Lösung von 4,9 g (0,025 Mol) 3,5-Dimethyl-4-hydroxymandelsäure und 3,0 g (0,075 Mol) Natriumhydroxid in 10 ml Wasser werden während ca. 15 Minuten 0,0375 Mol Dialkylsulfat zugetropft. Anschliessend wird das Reaktionsgemisch noch während einer Stunde bei 100 °C weiter gerührt. Nach dem Abkühlen wird mit konzentrierter Salzsäure angesäuert und das ausgefallene Produkt zweimal mit je ca. 30 ml Ethylacetat extrahiert. Die organischen Phasen werden mit Wasser gewaschen, vereinigt, über Natriumsulfat getrocknet und am Vakuumrotationsverdampfer eingeengt. Es resultieren die 4-Alkoxy-3,5-dimethyl-mandelsäuren, wie z.B. 3,5-Dimethyl-4-methoxy-mandelsäure, Smp. 134-136 °C (83 %) oder 4-Ethoxy-3,5-dimethyl-mandelsäure, Harz (81 %).

Herstellung von substituierten 4-Hydroxy-mandelsäuren:

0,30 Mol Ausgangsphenol werden in 150 ml 2N Natriumhydroxid-Lösung unter Stickstoffatmosphäre gelöst. Nach Abkühlen auf +5 °C werden 4,8 g (0,12 Mol) Natriumhydroxid und 13,3 ml (0,12 Mol) 50 %wässrige Glyoxylsäure zugegeben und das Reakionsgemisch während 4 Stunden bei Raumtemperatur gerührt. Nach jeweils 4 Stunden werden zweimal weitere 0,12 Mol Natriumhydroxid und Glyoxylsäure zugegeben (total 0,36 Mol). Das Reaktionsgemisch wird anschliessend noch 12 Stunden gerührt, dann mit konzentrierter Salzsäure neutralisiert und mit zweimal 75 ml Petrolether gewaschen. Die wässrige Phase

wird nun mit konzentrierter Salzsäure angesäuert und mit Ether mehrmals extrahiert. Die organischen Phasen werden vereinigt, über Magnesiumsulfat getrocknet und am Vakuumrotationsverdampfer eingeengt. Es werden so die folgenden Produkte erhalten: 3,5-Dimethyl-4-hydroxy-mandelsäure, Smp. 132-135 °C (85 %); 4-Hydroxy-3-methyl-mandelsäure, Smp. 115-120 °C, (55 %); 4-Hydroxy-3-tert.-butyl-mandelsäure, Smp. 156-158 °C, (26 %); und 3-Isopropyl-4-hydroxy-2-methyl-mandelsäure, Smp. 114-119 °C, (20%).

Beispiel 2: Herstellung von 2-(2-Allyloxy-5-tert.-butyl-phenyl)-2-phenylessigsäure (Verbindung (105), Tabelle 1).

Eine Suspension von 18,4 g (57,0 mMol) 2-(2-Allyloxy-5-tert.-buty-phenyl)-2-phenylessigsäureamid (Verbindung (101), Tabelle 1, Beispiel 1) und 30 g (0,54 Mol) Kaliumhydroxid in 70 ml Methanol wird während 57 Stunden unter Rückfluss gekocht. Anschliessend wird mit 150 ml Wasser verdünnt und das Methanol am Vakuumrotationsverdampfer eingeengt. Der wässrige Rückstand wird in eine Lösung von 20 ml konzentrierter Schwefelsäure in 500 ml Wasser gegossen. Das ausgefallene Produkt wird mit Dichlormethan extrahiert Die organischen Phasen werden mit Wasser gewaschen, vereinigt über Magnesiumsulfat getrocknet und am Vakuumrotationsverdampfer eingeengt. Kristallisation des Rückstandes aus Ligroin liefert 15,3 g (83 %) 2-(2-Allyloxy-5-tert.-butyl-phenyl)-2-phenylessigsäure, Smp. 120-122 °C (Verbindung (105), Tabelle 1).

In Analogie zu Beispiel 2 wird ausgehend von Verbindung (109) die Verbindung (110) erhalten.

Beispiel 3: Herstellung von 2-(2-Allyloxy-5-tert.-butyl-phenyl)-2-phenylessigsäurestearylester (Verbindung (106), Tabelle 1).

Eine Lösung von 3,24 g (10,0 mMol) 2-(2-Allyloxy-5-tert.-butyl-phenyl)-2-phenylessigsäure (Verbindung (105), Beispiel 2) und 2,70 g (10,0 mMol) Stearylalkohol (1-Octadecanol) in 25 ml Dichlormethan wird mit 100 mg (0,82 mMol) 4-Dimethylaminopyridin und 2,20 g (10,7 mMol) N,N'-Dicyclohexylcarbodiimid versetzt und während 60 Stunden bei Raumtemperatur gerührt. Das Reaktionsgemisch wird filtriert und am Vakuumrotationsverdampfer eingeengt. Chromatographie des Rückstandes an Kieslegel mit dem Laufmittelsystem Dichlormethan/Hexan = 1:1 liefert 4,0 g (69 %) 2-(2-Allyloxy-5-tert.-butyl-phenyl)-2-phenylessigsäurestearylester (Verbindung (106), Tabelle 1) als Oel.

In Analogie zu Beispiel 3 wird ausgehend von einem halben Aequivalent 1,2-Dodecandiol anstelle von Stearylalkohol die Verbindung (107) hergestellt.

Tabelle 1:

| Nr. | Verbindung | Smp. (°C) | C (%), H (%), N(%) (berechnet/gefunden) | Ausbeute (%) |
|---|---|---|---|---|
| 101 | 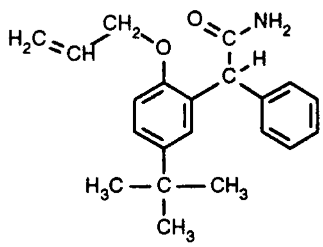 | 172-174 | 77,98 7,79 4,33<br>77,61 7,78 4,22 | 83 |
| 102 | 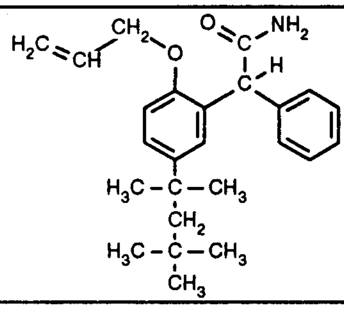 | 75-78 | 79,11 8,76 3,69<br>78,90 8,96 3,43 | 87 |
| 103 | 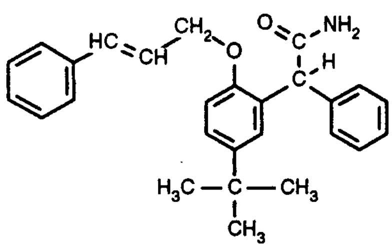 | 165-168 | 81,17 7,32 3,51<br>81,16 7,34 3,34 | 86 |
| 104 | 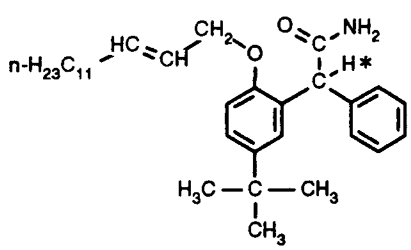 | Wachs | Charakterisiert durch $^1$H-NMR in $CDCl_3$<br>δ(H*) = 5,32 ppm | 55 |

Tabelle 1: (Fortsetzung)

| Nr. | Verbindung | Smp. (°C) | C (%), H (%), N(%) (berechnet/gefunden) | Ausbeute (%) |
|---|---|---|---|---|
| 105 | 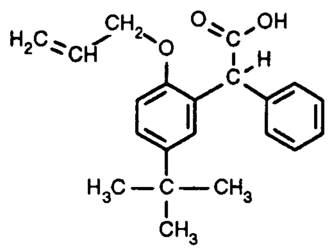 | 120-122 | 77,75 7,46 -<br>77,59 7,58 - | 83 |
| 106 | 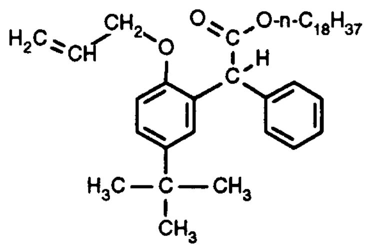 | Oel | 81,20 10,48 -<br>81,10 10,64 - | 69 |
| 107 | 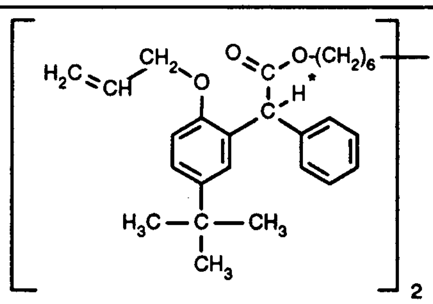 | Oel | Charakterisiert durch $^{1}$H-NMR in $CDCl_3$<br>$\delta(H^*) = 5{,}34$ ppm | 69 |
| 108 | 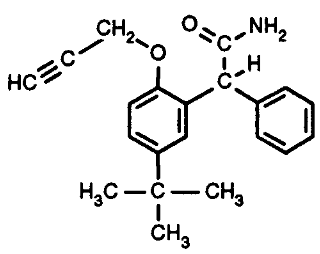 | 135-138 | 78,47 7,21 4,36<br>78,38 7,35 4,10 | 86 |

Tabelle 1: (Fortsetzung)

| Nr. | Verbindung | Smp. (°C) | C (%), H (%), N(%) (berechnet/gefunden) | Ausbeute (%) |
|---|---|---|---|---|
| 109 | 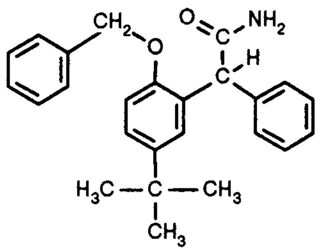 | 154-156 | 80,40 7,29 3,75<br>80,17 7,24 3,63 | 66 |
| 110 | 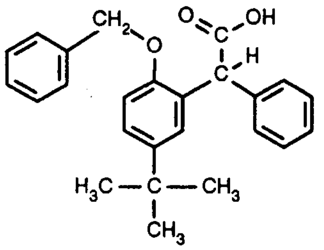 | 124-127 | 80,18 7,00 -<br>80,20 6,91 - | 75 |
| 111 | 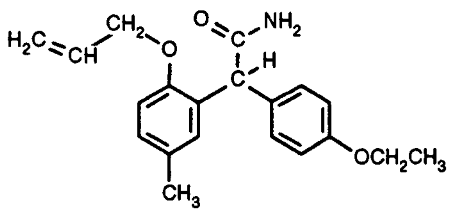 | 117-119 | 73,82 7,12 4,30<br>73,89 7,01 4,19 | 81 |
| 112 | 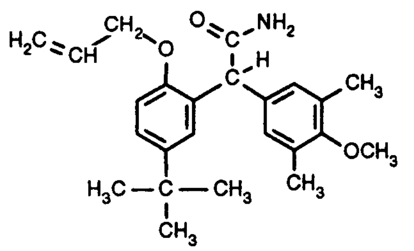 | 101-103 | 75,56 8,19 3,67<br>75,53 8,27 3,51 | 88 |

Beispiel 4: Stabilisierung von Polypropylen bei Mehrfachextrusion

1,3 kg Polypropylenpulver (Profax 6501)(Schmelzindex 3,4 g/10 min., gemessen bei 230 °C mit 2,16 kg) werden mit 0,05% Calciumstearat, 0,05% β-(3,5-Di-tert.butyl-4-hydroxyphenyl)-propionsäurepentaerythritester Irganox® 1010 (Ciba-Geigy) und 0,05% der Verbindung aus der Tabelle 1 vermischt. Diese Mischung wird in einem Extruder mit einem Zylinderdurchmesser von 20 mm und einer Länge von 400 mm mit 100 U/min extrudiert, wobei die drei Heizzonen auf die folgenden Temperaturen eingestellt werden: 260 °C, 270 °C und 280 °C. Das Extrudat wird zur Kühlung durch ein Wasserbad gezogen und anschließend granuliert. Nach zwei weiteren, in gleicher Weise verlaufenden Extrusionen wird der Schmelzindex gemessen (bei 230 °C mit 2,16 kg). Große Zunahme des Schmelzindex bedeutet starken Kettenabbau, also

schlechte Stabilität.

Die Ergebnisse sind in der folgenden Tabelle 2 zusammengefaßt:

Tabelle 2

| Verbindung aus Tabelle 1 | Schmelzindex nach 3 Extrusionen |
|---|---|
| — | 23,0 |
| 101 | 6,0 |
| 102 | 6,3 |
| 103 | 6,0 |
| 104 | 6,3 |
| 105 | 5,0 |
| 106 | 6,5 |
| 107 | 8,3 |
| 108 | 8,6 |

## Patentansprüche

**1.** Verbindungen der Formel I,

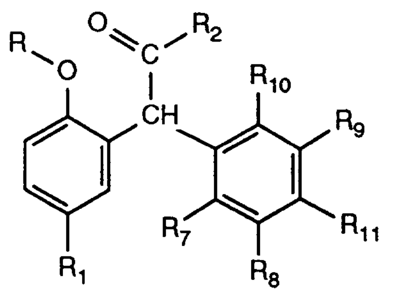

(I)

worin R unsubstituiertes oder mit Phenyl substituiertes $C_3$-$C_{18}$-2-Alkenyl, $C_3$-$C_8$-2-Alkinyl oder Phenyl-$C_1$-$C_4$-alkyl ist,
$R_1$ Wasserstoff, $C_1$-$C_{18}$-Alkyl, $C_5$-$C_{12}$-Cycloalkyl oder Phenyl-$C_1$-$C_4$-alkyl darstellt,
$R_2$ -$OR_4$ oder -$NR_5R_6$ ist,
$R_4$ Wasserstoff, $C_1$-$C_{18}$-Alkyl oder $(-CH_2-)_nG$ darstellt,
$R_5$ und $R_6$ unabhängig voneinander Wasserstoff, $C_1$-$C_{12}$-Alkyl oder Phenyl-$C_1$-$C_4$-alkyl bedeuten,
$R_7$, $R_8$, $R_9$ und $R_{10}$ unabhängig voneinander Wasserstoff, $C_1$-$C_4$-Alkyl oder $C_1$-$C_4$-Alkoxy darstellen, mit der Bedingung, dass mindestens einer der Reste $R_7$, $R_8$, $R_9$ oder $R_{10}$ Wasserstoff ist,
$R_{11}$ Wasserstoff, $C_1$-$C_{18}$-Alkoxy, $C_5$-$C_8$-Cycloalkoxy, Phenyl-$C_1$-$C_4$-alkoxy oder $C_3$-$C_{18}$-2-Alkenyloxy bedeutet,
n eine Zahl von 2 bis 12 ist, und
G eine Gruppe der Formel

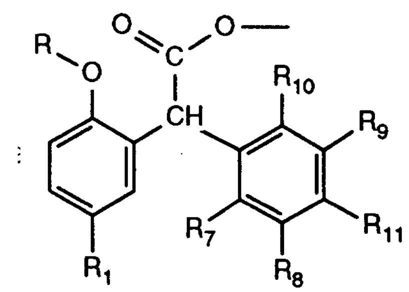

darstellt.

2. Verbindungen gemäss Anspruch 1, worin $R_7$, $R_8$, $R_9$, $R_{10}$ und $R_{11}$ Wasserstoff sind.

3. Verbindungen gemäss Anspruch 1, worin
R $-CH_2-CH=CH_2$, $-CH_2-C(CH_3)=CH_2$, $-CH_2-CH=CHR_3$, $-CH_2-C\equiv CH$ oder Phenyl-$C_1$-$C_4$-alkyl ist,
$R_1$ Wasserstoff oder $C_1$-$C_8$-Alkyl bedeutet,
$R_2$ $-OR_4$ oder $-NHR_5$ ist,
$R_3$ Phenyl, $C_1$-$C_{12}$-Alkyl, und
$R_5$ Wasserstoff, $C_1$-$C_4$-Alkyl oder Benzyl bedeutet, und
$R_{11}$ Wasserstoff, $C_1$-$C_{18}$-Alkoxy, $C_3$-$C_4$-2-Alkenyloxy oder Benzyloxy bedeutet.

4. Verbindungen gemäss Anspruch 1, worin
$R_1$ Methyl, t-Butyl oder $-C(CH_3)_2-CH_2-C(CH_3)_3$ ist.

5. Verbindungen gemäss Anspruch 1, worin $R_7$ und $R_{10}$ Wasserstoff sind.

6. Verbindungen gemäss Anspruch 1, worin
R unsubstituiertes oder mit Phenyl substituiertes $C_3$-$C_{14}$-2-Alkenyl, $-CH_2-C\equiv CH$ oder Benzyl bedeutet,
$R_1$ Wasserstoff oder $C_1$-$C_8$-Alkyl darstellt,
$R_2$ $OR_4$ oder $NH_2$ ist,
$R_7$ und $R_{10}$ Wasserstoff bedeuten,
$R_8$ und $R_9$ unabhängig voneinander Wasserstoff oder $C_1$-$C_4$-Alkyl darstellen, und
$R_{11}$ Wasserstoff oder $C_1$-$C_4$-Alkoxy bedeutet.

7. Zusammensetzung enthaltend
a) ein dem oxidativen, thermischen oder lichtinduzierten Abbau unterworfenes organisches Material und
b) mindestens eine Verbindung der Formel I gemäss Anspruch 1.

8. Zusammensetzung gemäss Anspruch 7, worin die Komponente a) ein synthetisches Polymer ist.

9. Zusammensetzung gemäss Anspruch 7, worin die Komponente b) in einer Menge von 0,0005 bis 5 % bezogen auf das Gewicht der Komponente a) vorliegt.

10. Zusammensetzungen gemäss Anspruch 7, enthaltend zusätzlich ein organisches Phosphit oder Phosphonit.

11. Verwendung der Verbindungen der in Anspruch 1 definierten Formel I als Stabilisatoren für organische Materialien gegen oxidativen, thermischen oder lichtinduzierten Abbau.

12. Verfahren zum Stabilisieren eines organischen Materials gegen oxidativen, thermischen oder lichtinduzierten Abbau, dadurch gekennzeichnet, dass man diesem mindestens eine Verbindung der in Anspruch 1 definierten Formel I einverleibt oder auf dieses aufbringt.

## Claims

1. A compound of the formula I

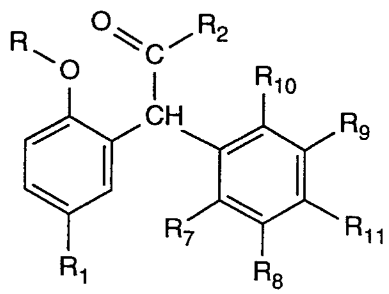

(I)

in which R is $C_3$-$C_{18}$-2-alkenyl, $C_3$-$C_8$-2-alkynyl or phenyl-$C_1$-$C_4$alkyl, each of which is unsubstituted or substituted by phenyl,
$R_1$ is hydrogen, $C_1$-$C_{18}$alkyl, $C_5$-$C_{12}$cycloalkyl or phenyl-$C_1$-$C_4$alkyl,
$R_2$ is $-OR_4$ or $-NR_5R_6$,
$R_4$ is hydrogen, $C_1$-$C_{18}$alkyl or $(-CH_2-)_nG$,
$R_5$ and $R_6$ independently of one another are hydrogen, $C_1$-$C_{12}$alkyl or phenyl-$C_1$-$C_4$alkyl,
$R_7$, $R_8$, $R_9$ and $R_{10}$ independently of one another are hydrogen, $C_1$-$C_4$alkyl or $C_1$-$C_4$alkoxy, with the proviso that at least one of the radicals $R_7$, $R_8$, $R_9$ or $R_{10}$ is hydrogen,
$R_{11}$ is hydrogen, $C_1$-$C_{18}$alkoxy, $C_5$-$C_8$cycloalkoxy, phenyl-$C_1$-$C_4$alkoxy or $c_3$-$C_{18}$-2-alkenyloxy,
n is a number from 2 to 12, and
G is a group of the formula

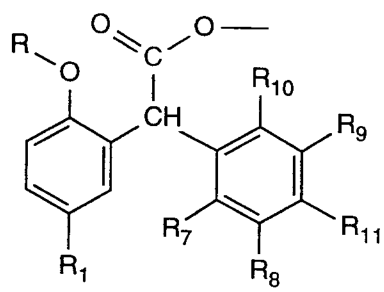

2. A compound according to claim 1, in which $R_7$, $R_8$, $R_9$, $R_{10}$ and $R_{11}$ are hydrogen.

3. A compound according to claim 1, in which R is $-CH_2-CH=CH_2$, $-CH_2-C(CH_3)=CH_2$, $-CH_2-CH=CHR_3$, $-CH_2-C{\equiv}CH$ or phenyl-$C_1$-$C_4$alkyl,
$R_1$ is hydrogen or $C_1$-$C_8$alkyl,
$R_2$ is $-OR_4$ or $-NHR_5$,
$R_3$ is phenyl, $C_1$-$C_{12}$alkyl, and
$R_5$ is hydrogen, $C_1$-$C_4$alkyl or benzyl, and
$R_{11}$ is hydrogen, $C_1$-$C_{18}$alkoxy, $C_3$-$C_4$-2-alkenyloxy or benzyloxy.

4. A compound according to claim 1, in which
$R_1$ is methyl, t-butyl or $-C(CH_3)_2-CH_2-C(CH_3)_3$.

5. A compound according to claim 1, in which $R_7$ and $R_{10}$ are hydrogen.

6. A compound according to claim 1, in which
R is unsubstituted or phenyl-substituted $C_3$-$C_{14}$-2-alkenyl, $-CH_2-C{\equiv}CH$ or benzyl,
$R_1$ is hydrogen or $C_1$-$C_8$alkyl,
$R_2$ is $OR_4$ or $NH_2$,

$R_7$ and $R_{10}$ are hydrogen,
$R_8$ and $R_9$ independently of one another are hydrogen or $C_1$-$C_4$alkyl, and
$R_{11}$ is hydrogen or $C_1$-$C_4$alkoxy.

**7.** A composition comprising
a) an organic material subjected to oxidative, thermal or light-induced degradation, and
b) at least one compound of the formula I according to claim 1.

**8.** A composition according to claim 7, in which component a) is a synthetic polymer.

**9.** A composition according to claim 7, in which component b) is present in an amount from 0.0005 to 5 % based on the weight of component a).

**10.** A composition according to claim 7, additionally comprising an organic phosphite or phosphonite.

**11.** The use of a compound of the formula I defined in claim 1 as stabilisers for organic material against oxidative, thermal or light-induced degradation.

**12.** A process for stabilising an organic material against oxidative, thermal or light-induced degradation, which comprises incorporating or applying at least one compound of the formula I defined in claim 1 into, or onto, respectively, this organic material.

**Revendications**

**1.** Composés de formule (I)

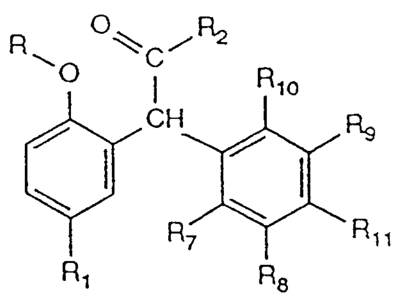

(I)

dans laquelle

| | |
|---|---|
| R | est un phényl-alkyle en $C_1$-$C_4$, un 2-alcynyle en $C_3$-$C_8$, ou un 2-alcényle en $C_3$-$C_{18}$, non substitué ou phénylsubstitué, |
| $R_1$ | représente l'hydrogène, un alkyle en $C_1$-$C_{18}$, un cycloalkyle en $C_5$-$C_{12}$ ou un phényl-alkyle en $C_1$-$C_4$, |
| $R_2$ | est $-OR_4$ ou $-NR_5R_6$, |
| $R_4$ | représente l'hydrogène, un alkyle en $C_1$-$C_{18}$ ou $(-CH_2-)_nG$, |
| $R_5$ | et $R_6$ signifient, indépendamment l'un de l'autre, l'hydrogène, un alkyle en $C_1$-$C_{12}$ ou un phényl-alkyle en $C_1$-$C_4$, |
| $R_7$, | $R_8$, $R_9$ et $R_{10}$ représentent indépendamment l'un de l'autre, un hydrogène, un alkyle en $C_1$-$C_4$ ou un alcoxy en $C_1$-$C_4$, avec la condition qu'au moins un des restes $R_7$, $R_8$, $R_9$ ou $R_{10}$ soit un hydrogène, |
| $R_{11}$ | signifie un hydrogène, un alcoxy en $C_1$-$C_{18}$, un cycloalcoxy en $C_5$-$C_8$, un phényl-alcoxy en $C_1$-$C_4$ ou un 2-alcényloxy en $C_3$-$C_{18}$, |
| n | est un nombre allant de 2 à 12, et |
| G | représente un groupe de formule |

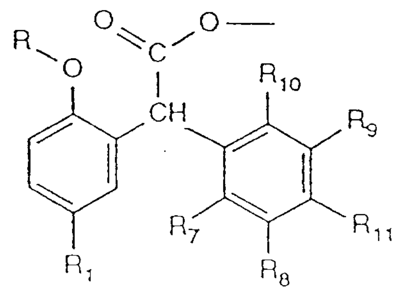

2. Composés selon la revendication 1, dans lesquels $R_7$, $R_8$, $R_9$, $R_{10}$ et $R_{11}$ sont des hydrogènes.

3. Composés selon la revendication 1, dans lesquels
   - R est choisi parmi les restes $-CH_2-CH=CH_2$, $-CH_2-C(CH_3)=CH_2$, $-CH_2-CH=CHR_3$, $-CH_2-C\equiv CH$ ou phénylalkyle en $C_1$-$C_4$,
   - $R_1$ signifie l'hydrogène ou un alkyle en $C_1$-$C_8$,
   - $R_2$ est $-OR_4$ ou $-NHR_5$,
   - $R_3$ signifie le phényle, un alkyle en $C_1$-$C_{12}$, et
   - $R_5$ signifie un hydrogène, un alkyle en $C_1$-$C_4$ ou un benzyle, et
   - R11 signifie un hydrogène, un alcoxy en $C_1$-$C_{18}$, un 2-alcényloxy en $C_3$-$C_4$ ou un benzyloxy.

4. Composés selon la revendication 1, dans lesquels
   - $R_1$ est le méthyle, le t-butyle ou $-C(CH_3)_2-CH_2-C(CH_3)_3$.

5. Composés selon la revendication 1, dans lesquels $R_7$ et $R_{10}$ sont des hydrogènes.

6. Composés selon la revendication 1, dans lesquels
   - R signifie les $-CH2-C\equiv CH$ ou bien benzyle, 2-alcényle en $C_3$-$C_{14}$ non substitués ou bien phényl-substitués,
   - $R_1$ représente un hydrogène ou un alkyle en $C_1$-$C_8$,
   - $R_2$ est $-OR_4$ ou $-NH_2$,
   - $R_7$ et $R_{10}$ signifient un hydrogène, $R_8$ et $R_9$ représentent, indépendamment l'un de l'autre, un hydrogène ou un alkyle en $C_1$-$C_4$, et
   - R11 signifie un hydrogène ou un alcoxy en $C_1$-$C_4$.

7. Composition contenant
   a) une matière organique soumise à des dégradations oxydantes, thermiques ou induites par la lumière et
   b) au moins un composé de formule I selon la revendication 1.

8. Composition selon la revendication 7, dans laquelle le composant a) est un polymère synthétique.

9. Composition selon la revendication 7, dans laquelle le composant b) se trouve dans une quantité comprise entre 0,0005 et 5% par rapport au poids du composant a).

10. Compositions selon la revendication 7, contenant en plus un phosphite ou un phosphonite organique.

11. Utilisation des composés de formule I définis dans la revendication 1 en tant que stabilisants pour des matières organiques contre les dégradations oxydantes, thermiques, ou induites par la lumière.

12. Procédé pour stabiliser une matière organique contre les dégradations oxydantes, thermiques ou induites par la lumière, caractérisé en ce qu'on lui incorpore au moins un composé de formule I défini dans la revendication 1 ou bien on le dépose sur celle-ci.